# EUROPEAN PATENT APPLICATION

(11) **EP 0 887 593 A1**
(43) Date of publication of application: **30.12.1998**
(21) Application number: 97310682.6
(22) Date of filing: 30.12.1997
(51) Int. Cl.: F24C 15/00

(54) **Microwave oven with antibacterial coating**

(30) Priority: 27.06.1997 KR 9728368
(71) Applicant: Samsung Electronics Co., Ltd., Suwon City, Kyungki-do (KR)
(72) Inventor: Kim, Hyang-Gi, Suwon-City, Kyungki-do (KR)
(74) Representative: Geary, Stuart Lloyd

(57) **Abstract**

The walls of the cooking chamber (20) of a microwave oven are coated with an antibacterial/antifungal agent to prevent the growth of harmful organisms. The preferred coating comprises a zeolite and silver.

## Description

The present invention relates to a microwave oven including a cooking chamber.

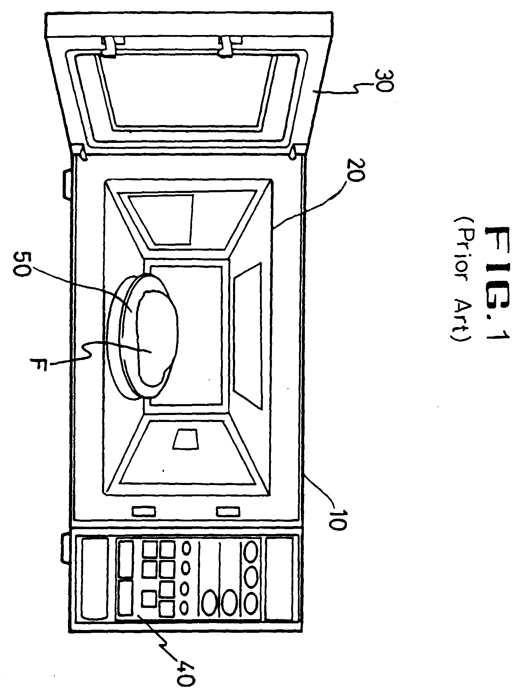

Referring to Figure 1, a known microwave oven comprises a body 10, a cooking chamber 20 within the body 10 and offset to one side, a door 30 providing access to the cooking chamber 20, a control panel 40 located on the front of the body 10 beside the door 30, and a turntable 50 at the bottom of the cooking chamber 20.

During cooking, food F in the cooking chamber 20 may boil over or splatter making the inside of the cooking chamber 20 dirty. As a result, eumycetes and bacteria find a place to breed within the cooking chamber 20.

It is an aim of the present invention to ameliorate the aforementioned problem.

A microwave oven according to the present invention is characterised in that the internal surfaces of the cooking chamber are coated with an antibacterial and/or antifungal material.

Preferably, the material includes silver.

Preferably, the material includes a zeolite.

In addition to the walls of the cooking chamber itself, items located in the cooking chamber, such as a food-supporting turntable can also be coated with antibacterial and/or antifungal material.

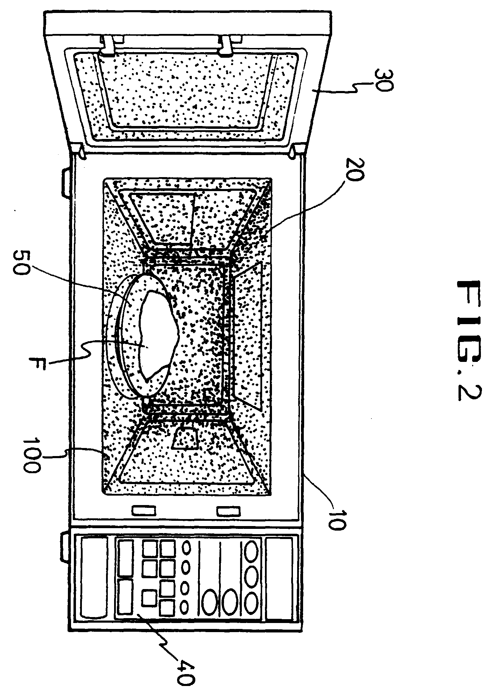

An embodiment of the present invention will now be described, by way of example, with reference to Figure 2 of the accompanying drawings, in which:
Figure 1 shows a known microwave oven; and
Figure 2 shows a microwave oven according to the present invention.

Referring to Figure 2, an antibacterial/antifungicidal coating 100 is formed on each of the internal walls of the cooking chamber 20, including the inward facing wall of the door 30. The antibacterial/antifungicidal coating 100 also extends over a turntable 50 in the cooking chamber 20. The coating 100 is effective for killing bacteria that would otherwise grow in food that has escaped from cooking vessels.

The coating 100 comprises a combination of a zeolite and silver (zeolite-Ag) and is also effective prevent the propagation of green mold. Zeolite-Ag is heat resistant and does not discolour even when heated to 300°C. Furthermore, it is stable in the presence of water and organic solvents and remains effective for a long time.

The layer 100 has been found to ball escherichia coli, salmonella typhimurium, pseudomanas aeruginaosa, and bacillus alvei and prevent the spread of green mold even under conditions of high temperature and humidity.

Escherichia coli are non-pathological microbes, the type species of the genus, that naturally occur amongst the flora in part of the intestines of vertebrates. Escherichia coli is used as a contamination index for checking food hygiene.

Food poisoning results from the ingestion of food contaminated with bacteria or toxins produced by bacteria, particularly when the weather is hot, such as in summer.

Salmonella is an enterobacterium that is pathogenic for humans and other warm-blooded animals and causes food poisoning, gastrointestinal inflammation, typhoid fever, and septicemia

Bacillus alvei is one of the aerobic rod-shaped gram-positive bacteria producing endospores that do not thicken the rod and includes many saphrophytes and some parasites. They exist in the soil, hay, brooks, the surface of soybean paste or soy sauce, milk. They curdle milk, break starch into simple sugars, and resolve oil and fat. They quickly propagate themselves by fission and are strongly resistant to temperature, preventing conjunctivitis.

The following results were obtained using a microwave oven according to the present invention.

Samples coated with antibiotic material according to the present invention and samples of conventional microwave ovens, a control group, were prepared in 10cm x 10cm.

*Escherichia coli* KCTC1116, *Pseudomonas aeruginosa* KCTC1750, and *Salmonella typhimurium* KCTC1926 were used as test strains.

The above three test strains, each stored in a slope culture, were inoculated into 10ml liquid medium containing the substances listed in table 1 and were cultivated in this liquid medium at 37°C for 18 hours, to obtain a preculture fluid.

The preculture fluid was diluted with a sterilized phosphate buffer solution (0.2 mole, pH7), so that 10³ to 10⁴ of each strain were contained in 10ml liquid medium. 90ml phosphate buffer solution (0.2 mole, pH7) was put in a separate 250ml triangular flask as a test liquid.

70ml buffer solution for cultivation was separated in a 250ml triangular flask, and 5ml of the test liquid was separated. The above samples were each added to the test strains to make test culture liquids. After shaking and mixing the test culture liquids well, 10⁻², 10⁻² solutions were made as a buffer solution for dilution with 1ml portion of the respective test culture liquids. 1ml of each buffer solution was mixed with 20ml of medium containing substances listed in table 2 in a petri dish, and medium was then hardened. Each solution was cultivated in the culture medium at 37°C for 24 hours, and the number of colonies of each test strain was counted and this number was used to calculate back by the multiple of dilution. The resultant value was the initial number.

The test culture liquids were shake-cultured in a water bath at 30°C agitated at 140rpm. After 24 hours, 10⁻¹, 10⁻², and 10⁻³ solutions were made as a buffer solution for dilution with a 1ml portion of the respective test culture liquids. Using the above colony counting method, the number of colonies of each petri dish was counted, and this number was used to calculate back by the multiple of dilution. The resultant value was the number of colonies cultivated per hour.

Table 3 shows the result from the test conducted to compare the inventive microwave oven with the control group of the conventional microwave ovens in antibiotic effect.

### [Nutrient Broth]

**Table 1**

| Ingredient | Content |
|---|---|
| Beef extract | 3g |
| Peptone | 5g |
| Distilled water | 100ml (pH7) |

### [Nutrient Broth]

**Table 2**

| Ingredient | Content |
|---|---|
| Beef extract | 3g |
| Peptone | 5g |
| Glucose (or dextrose) | 1g |
| Agar | 15g |
| Distilled water | 100ml (pH7.2) |

**Table 3**

| Bacterium | Sample | Initial number | After 24 hours |
|---|---|---|---|
| Escherichia coli | Control group | 3.42x10⁵ | 6.80x10⁵ |
| | This invention | 3042x10⁵ | <1 |
| Pseudomonas aeruginosa | Control group | 7.50x10⁵ | 2.20x10⁴ |
| | This invention | 7.50x10⁵ | <1 |
| Salmonella typhimurium | Control group | 5.20x10⁵ | 5.90x10⁵ |
| | This invention | 5.20x10⁵ | <1 |

This microwave oven has an antibacterial effect superior to the control group's as shown in Table 3.

As described above, the antibiotic microwave oven of the present invention includes an antibacterial layer formed on each of the inner walls of the cooking chamber, thus preventing foodstuffs cooked in the cooking chamber from being contaminated by various microbes.

It will be apparent to those skilled in the art that various modifications and variations cam be made in the antibiotic microwave oven of the present invention without departing from the spirit or scope of the invention.

## Claims

1. A microwave oven including a cooking chamber (20), **characterised in that** the internal surfaces of the cooking chamber (20) are coated with an antibacterial and/or antifungal material.

2. An oven according to claim 1, wherein said material includes silver.

3. An oven according to claim 1 or 2, wherein said material includes a zeolite.

4. An antibiotic microwave oven for cooking a foodstuff within its cooking chamber utilizing high frequency energy, comprising an antibiotic layer provided to the cooking chamber for killing bacteria bred within the cooking chamber contaminated by the foodstuff being cooked and for preventing the propagation of harmful microbes.

5. An antibiotic microwave oven according to claim 4, wherein the antibiotic layer is formed of zeolite-Ag, thus killing escherichia coli, salmonella typhimurium, pseudomanas aeruginaosa, and bacillus alvei.
